# EUROPEAN PATENT APPLICATION

(11) **EP 4 497 437 A1**
(43) Date of publication of application: **29.01.2025**
(21) Application number: 23774883.5
(22) Date of filing: 20.03.2023
(51) Int. Cl.: A61K 31/498, A61K 31/48, A61P 19/00, A61P 21/00, A61P 25/02

(54) **DRUG FOR TREATING OR PREVENTING CHARCOT-MARIE-TOOTH DISEASE**

(30) Priority: 22.03.2022 JP 2022045440
(71) Applicant: Kyoto University, Kyoto-shi, Kyoto 606-8501 (JP)
(72) Inventor: INOUE, Haruhisa, Kyoto-shi, Kyoto 606-8501 (JP); IMAMURA, Keiko, Kyoto-shi, Kyoto 606-8501 (JP)
(74) Representative: Hoffmann Eitle
(86) International application number: PCT/JP2023/010944
(87) International publication number: WO 2023/182288

(57) **Abstract**

The present invention provides a drug for treating or preventing Charcot-Marie-Tooth disease, said drug comprising a compound represented by formula (I) 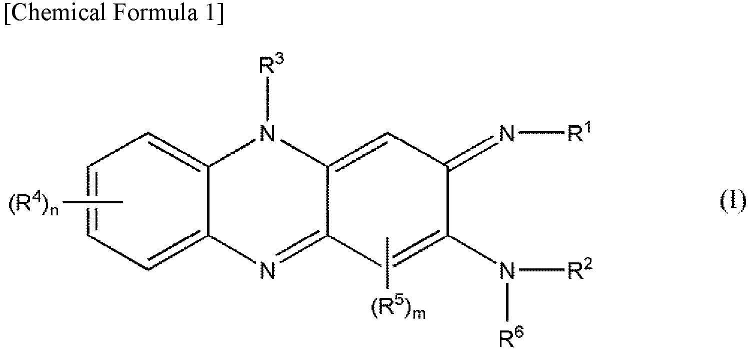 (in formula (I), the symbols are as defined in the attached description) or formula (II) 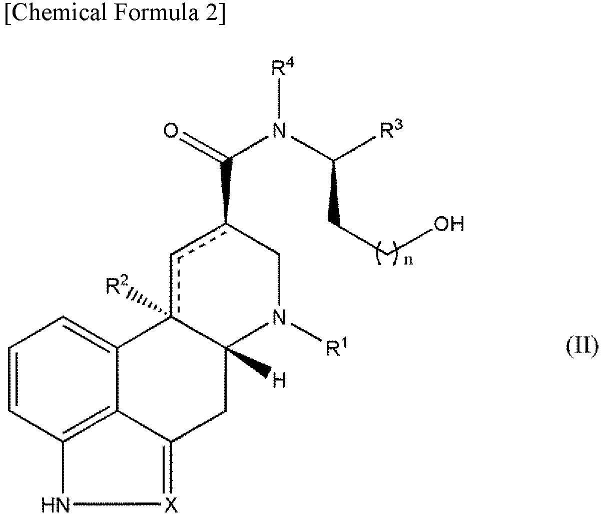 (in formula (II), the symbols are as defined in the attached description), or a salt of the compound.

## Description

### FIELD

The present invention relates to a therapeutic or prophylactic medicament for Charcot-Marie-Tooth disease. In particular, the present invention relates to a therapeutic or prophylactic medicament for Charcot-Marie-Tooth disease, comprising clofazimine or an analog thereof or methylergometrine or an analog thereof.

### BACKGROUND

Charcot-Marie-Tooth disease (CMT), which was reported by three persons, namely, Charcot, Marie, and Tooth, in 1886, is the most common peripheral nerve disease mainly caused by genetic abnormalities. CMT affects about one in 2,500 to 10,000 people worldwide. CMT causes slowly progressive muscle atrophy, weakness, sensory disorder, and foot deformity. At present, there is no effective fundamental therapeutic medicament for CMT.

CMT is broadly classified into axonal-type CMT (about 12%) and demyelinating-type CMT (about 75%) according to the diseased part, and the most frequent causative gene of the axonal-type CMT is mitofusin 2 (MFN2), a mitochondrial protein (for example, NPL 1). In addition to MFN2, mitofusin 1 (MFN1) is also an isoform of mitofusin. It has been reported that overexpression of MFN1 in transgenic mice in which a human MFN2 mutant (R94Q mutant) is expressed in a neuron-specific manner alleviates the symptoms of CMT (NPL 2).

It is considered that pathological conditions can be reproduced in vitro by establishing induced pluripotent stem cells (iPS cells) from cells derived from a patient using reprogramming technology and inducing the differentiation of these iPS cells into cells that cause the disease. Previously, the inventors of the present invention developed a model for evaluating the neurotoxicity of test substances by generating iPS cells from CMT patients having a mutation in the MFN2-encoding gene and then differentiating the iPS cells into neurons including motor neurons (PTL 1, NPL 3). Using such a model, the inventors made a comparative analysis of the phenotype between neurons derived from iPS cells of CMT patients (CMT-N) and neurons derived from iPS cells of healthy subjects (control), and found out that mitochondria in neurites of CMT-N were morphologically shorter and had lower mobility than those in the control.

Although the development of the neurotoxicity evaluation system using iPS cell-derived neurons as described above advances the discovery of promising candidate substances (seeds of therapeutic medicaments), there would still be a long way to go before they are put into practical application as pharmaceuticals.

Meanwhile, drug repositioning (DR) is a new research concept that is being discussed as a method for breaking through the current impasse in research for new drug development. DR is a strategy aimed at discovering new drug efficacies of existing drugs whose safety and pharmacokinetics in humans have already been confirmed by their proven results and putting them into practical application. A large amount of available existing data can reduce the cost required for development, and there are further advantages such as the presence of accumulated know-how and materials (neighboring compounds and the like).

### [CITATION LIST]

### [PATENT LITERATURE]

[PTL 1] International Patent Publication No. WO2018/207789

### [NON PATENT LITERATURE]

[NPL 1] Abe A. et al., J Hum Genet., 56(5): 364-368 (2011)
[NPL 2] Zhou Y. et al, J Clin Invest., 129(4): 1756-1771 (2019)
[NPL 3] Ohara R. et al., Clin Pharmacol Ther., 101(6): 754-762 (2017)

### SUMMARY

### [TECHNICAL PROBLEM]

In light of the foregoing, it is an object of the present invention to provide an agent having therapeutic or prophylactic activity for CMT and to accelerate the development of a therapeutic or prophylactic medicament for CMT as a practical pharmaceutical, through drug repositioning.

### [SOLUTION TO PROBLEM]

The inventors of the present invention induced differentiation of motor neurons from iPS cells established from patients having a mutation in the MFN2-encoding gene according to the method described in NPL 3, and using the survival of these motor neurons as an index, they performed screening of known compound libraries including drugs already put on the market as pharmaceuticals to search for compounds that increase the expression level of the MFN1 gene or the MFN2 gene. As a result of such screening, the inventors successfully identified the compounds that increase the expression level of the MFN1 gene or the MFN2 gene. The inventors conducted further research based on this finding, which led to completion of the present invention.

Specifically, the invention provides the following.
[1] A therapeutic or prophylactic medicament for Charcot-Marie-Tooth disease, comprising:
   a compound represented by the following formula (I) or (II), or a salt thereof: 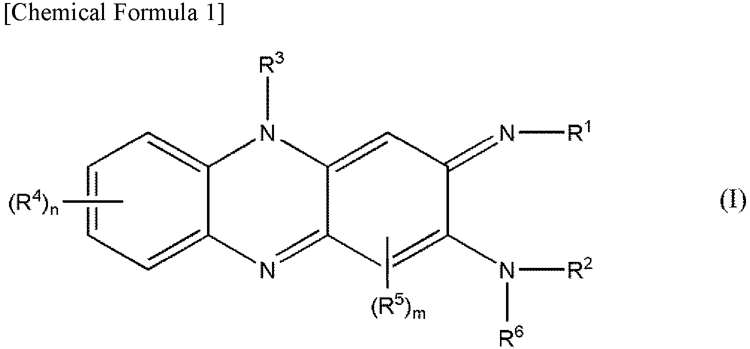 [in the formula (I),
   R¹ represents a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
   R² and R³ each independently represent a group that is selected from the group consisting of C₃-C₇ alicyclic hydrocarbon groups and 6- to 10-membered monocyclic or bicyclic aromatic groups, wherein each hydrogen atom in the group may be substituted and the group may have 1 to 4 heteroatoms each independently selected from nitrogen, oxygen, and sulfur;
   n and m are each independently an integer from 0 to 2;
   each R⁴ and each R⁵ independently represent a halogen atom, CN, or a C₁-C₃ aliphatic group in which each hydrogen atom may be substituted; and
   R⁶ represents a hydrogen atom or a C₁-C₃ aliphatic group in which each hydrogen atom may be substituted], 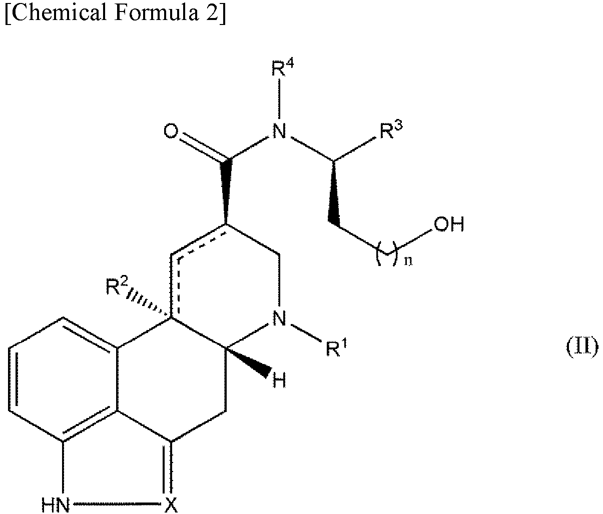 [in the formula (II),
      R¹ represents a hydrogen atom or a C₁-C₃ aliphatic group in which each hydrogen atom may be substituted;
      R² may or may not be present, and when present, R² represents a hydrogen atom;
      R³ and R⁴ each independently represent a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
      n is an integer from 0 to 2;
      X is a nitrogen atom or CZ [where Z is a hydrogen atom, a halogen atom, CN, or a C₁-C₃ aliphatic group in which each hydrogen atom may be substituted]; and
      a dotted line indicates the presence of a double bond at either position indicated by the dotted line].
[2-1] The therapeutic or prophylactic medicament according to [1], wherein R² and R³ in the formula (I) are each independently a 6- to 10-membered monocyclic aromatic group in which each hydrogen atom may be substituted and that may have 1 to 4 heteroatoms each independently selected from nitrogen, oxygen, and sulfur.
[2-2] The therapeutic or prophylactic medicament according to [1] or [2-1], wherein R² and R³ in the formula (I) are both 6- to 10-membered monocyclic aromatic groups in which each hydrogen atom may be substituted.
[2-3] The therapeutic or prophylactic medicament according to any one of [1] to [2-2], wherein R² and R³ in the formula (I) are both 6-membered monocyclic aromatic groups in which each hydrogen atom may be substituted.
[2-4] The therapeutic or prophylactic medicament according to any one of [1] to [2-3], wherein
   at least one of the aromatic groups represented by R² and R³ in the formula (I) is an aromatic group in which at least one hydrogen atom is substituted with a halogen atom.
[2-5] The therapeutic or prophylactic medicament according to [2-4], wherein the halogen atom is a chlorine atom.
[3-1] The therapeutic or prophylactic medicament according to any one of [1] to [2-5], wherein R¹ in the formula (I) is a C₁-C₆ aliphatic group or a C₃-C₇ alicyclic hydrocarbon group, wherein each hydrogen atom in the group may be substituted and the group may have 1 to 4 heteroatoms each independently selected from nitrogen, oxygen, and sulfur.
[3-2] The therapeutic or prophylactic medicament according to any one of [1] to [3-1], wherein R¹ in the formula (I) is a C₁-C₆ aliphatic group in which each hydrogen atom may be substituted.
[3-3] The therapeutic or prophylactic medicament according to any one of [1] to [3-2], wherein R¹ in the formula (I) is an aliphatic group in which each hydrogen atom is substituted with an a C₁-C₃ aliphatic group.
[3-4] The therapeutic or prophylactic medicament according to [3-3], wherein the C₁-C₃ aliphatic group is a C₁-C₃ alkyl group.
[4-1] The therapeutic or prophylactic medicament according to any one of [1] to [2-3] and [3-1], wherein the formula (I) is represented by the following formulae (I-1) to (I-10). 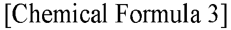 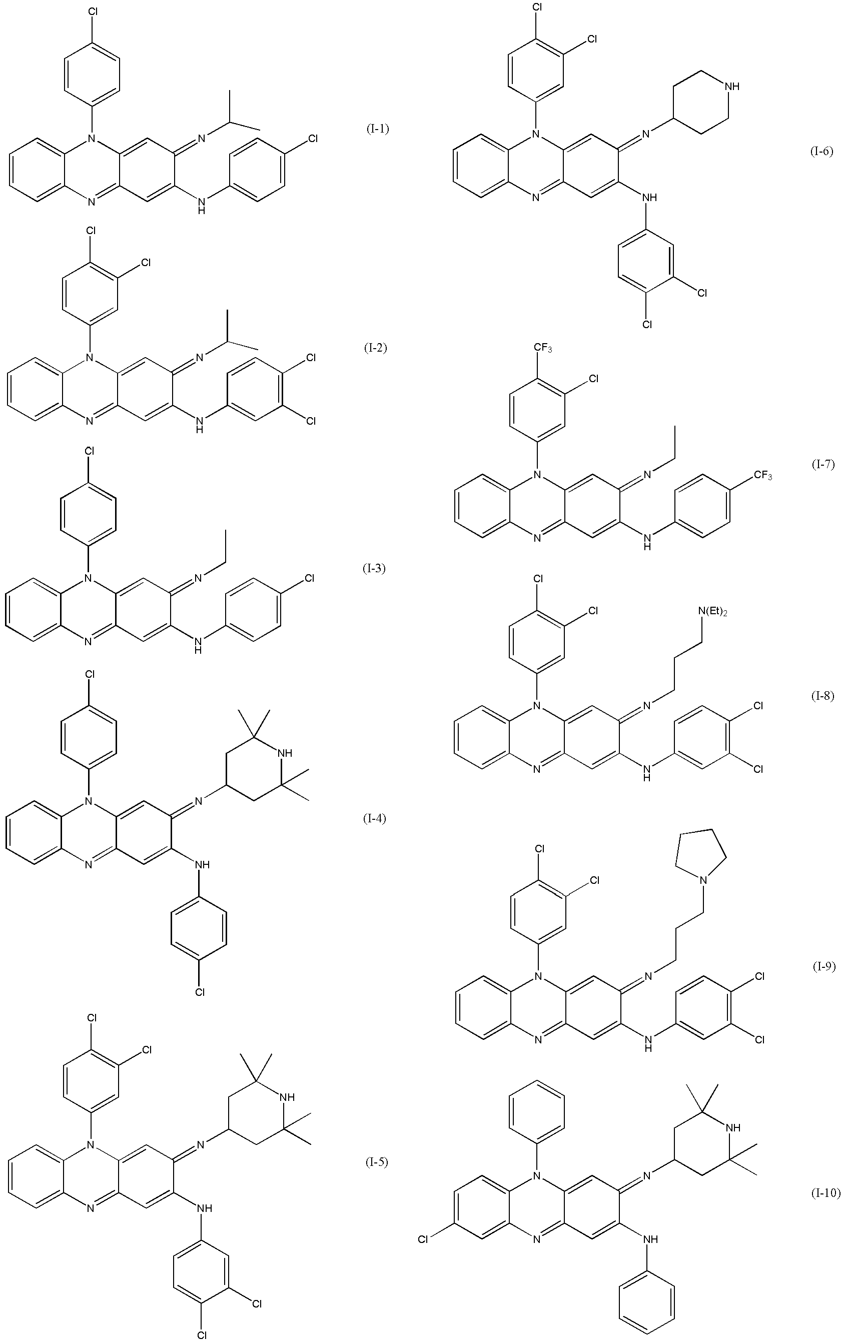
[4-2] The therapeutic or prophylactic medicament according to any one of [1] to [3-2], wherein the formula (I) is represented by the following formula (I-1). 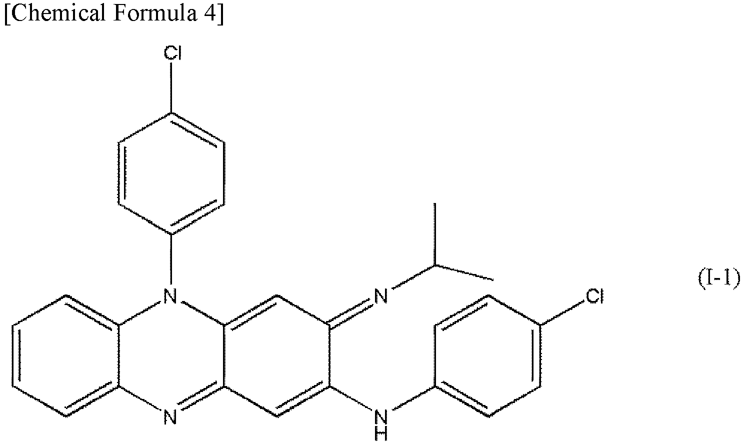
[5-1] The therapeutic or prophylactic medicament according to [1], wherein X in the formula (II) is CZ [where Z represents a hydrogen atom, a halogen atom, or CN].
[5-2] The therapeutic or prophylactic medicament according to [5-1], wherein Z is a hydrogen atom.
[6-1] The therapeutic or prophylactic medicament according to [1], [5-1], or [5-2], wherein R³ and R⁴ in the formula (II) are each independently a hydrogen atom or a C₁-C₃ aliphatic group in which each hydrogen atom may be substituted.
[6-2] The therapeutic or prophylactic medicament according to any one of [1] and [5-1] to [6-1], wherein R³ in the formula (II) is a C₁-C₃ aliphatic group.
[6-3] The therapeutic or prophylactic medicament according to [6-1] or [6-2], wherein at least one of the aliphatic groups is an alkyl group.
[6-4] The therapeutic or prophylactic medicament according to any one of [1] and [5-1] to [6-3], wherein R⁴ in the formula (II) is a hydrogen atom.
[7] The therapeutic or prophylactic medicament according to any one of [1] and [5-1] to [6-4], wherein the formula (II) is represented by the following formula (II-1). 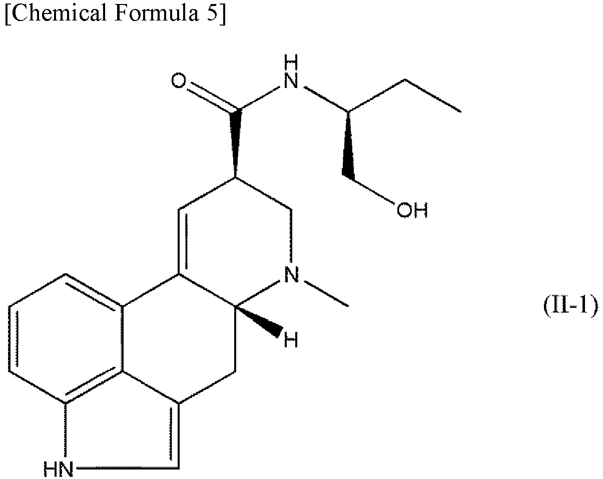
[8] The therapeutic or prophylactic medicament according to any one of [1] to [7], wherein the Charcot-Marie-Tooth disease is Charcot-Marie-Tooth disease type 2.
[9] An agent for promoting mitofusin 1 gene expression, comprising:
   a compound represented by the following formula (I) or (II), or a salt thereof: 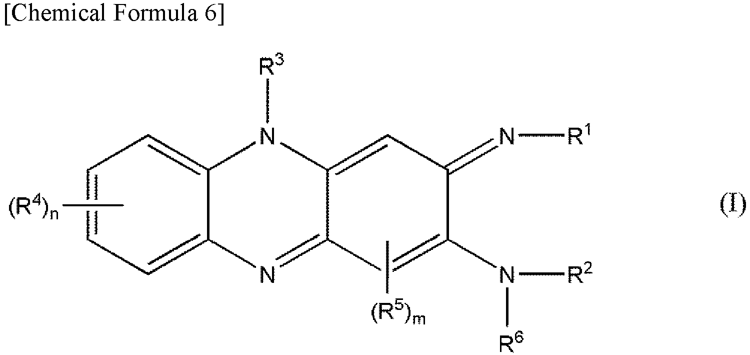 [in the formula (I),
   R¹ represents a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
   R² and R³ each independently represent a group that is selected from the group consisting of C₃-C₇ alicyclic hydrocarbon groups and 6- to 10-membered monocyclic or bicyclic aromatic groups, wherein each hydrogen atom in the group may be substituted and the group may have 1 to 4 heteroatoms each independently selected from nitrogen, oxygen, and sulfur;
   n and m are each independently an integer from 0 to 2;
   each R⁴ and each R⁵ independently represent a halogen atom, CN, or a C₁-C₃ aliphatic group in which each hydrogen atom may be substituted; and
   R⁶ represents a hydrogen atom or a C₁-C₃ aliphatic group in which each hydrogen atom may be substituted], 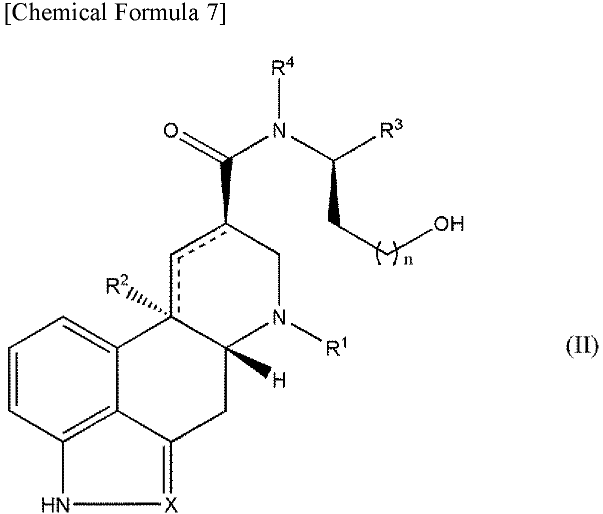 [in the formula (II),
      R¹ represents a hydrogen atom or a C₁-C₃ aliphatic group in which each hydrogen atom may be substituted;
      R² may or may not be present, and when present, R² represents a hydrogen atom;
      R³ and R⁴ each independently represent a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
      n is an integer from 0 to 2;
      X is a nitrogen atom or CZ [where Z is a hydrogen atom, a halogen atom, CN, or a C₁-C₃ aliphatic group in which each hydrogen atom may be substituted]; and
      a dotted line indicates the presence of a double bond at either position indicated by the dotted line].
[10] A method for treating or preventing Charcot-Marie-Tooth disease in a mammal, comprising: administering to the mammal an effective amount of a compound represented by the following formula (I) or (II), or a salt thereof: 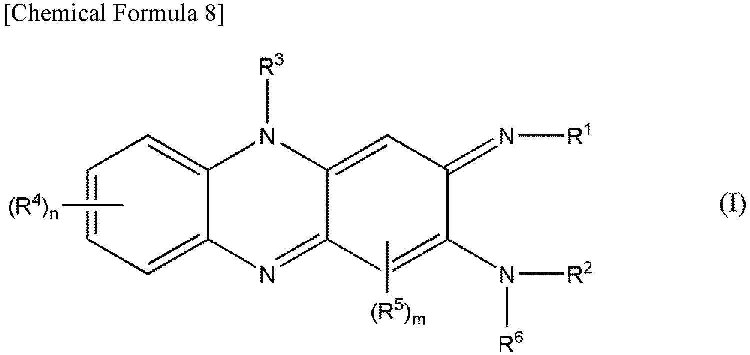 [in the formula (I),
   R¹ represents a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
   R² and R³ each independently represent a group that is selected from the group consisting of C₃-C₇ alicyclic hydrocarbon groups and 6- to 10-membered monocyclic or bicyclic aromatic groups, wherein each hydrogen atom in the group may be substituted and the group may have 1 to 4 heteroatoms each independently selected from nitrogen, oxygen, and sulfur;
   n and m are each independently an integer from 0 to 2;
   each R⁴ and each R⁵ independently represent a halogen atom, CN, or a C₁-C₃ aliphatic group in which each hydrogen atom may be substituted; and
   R⁶ represents a hydrogen atom or a C₁-C₃ aliphatic group in which each hydrogen atom may be substituted], 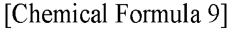 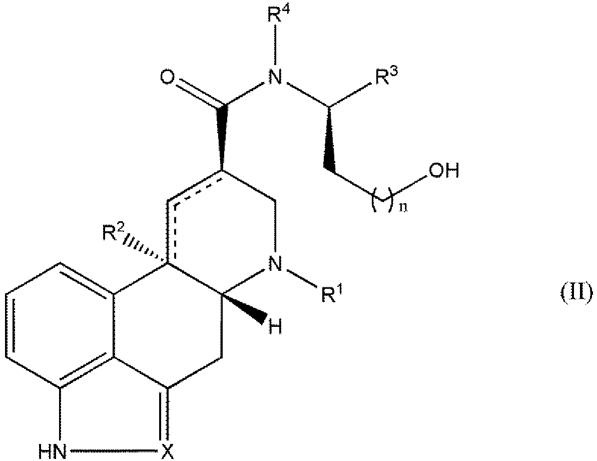 [in the formula (II),
      R¹ represents a hydrogen atom or a C₁-C₃ aliphatic group in which each hydrogen atom may be substituted;
      R² may or may not be present, and when present, R² represents a hydrogen atom;
      R³ and R⁴ each independently represent a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
      n is an integer from 0 to 2;
      X is a nitrogen atom or CZ [where Z is a hydrogen atom, a halogen atom, CN, or a C₁-C₃ aliphatic group in which each hydrogen atom may be substituted]; and
      a dotted line indicates the presence of a double bond at either position indicated by the dotted line].
[11] A compound represented by the following formula (I) or (II), or a salt thereof for use in treatment or prevention of Charcot-Marie-Tooth disease: 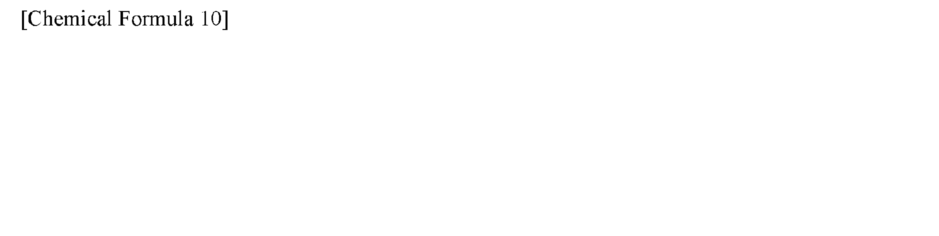 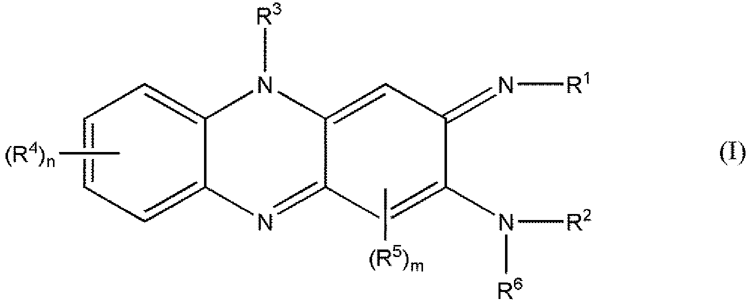 [in the formula (I),
   R¹ represents a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
   R² and R³ each independently represent a group that is selected from the group consisting of C₃-C₇ alicyclic hydrocarbon groups and 6- to 10-membered monocyclic or bicyclic aromatic groups, wherein each hydrogen atom in the group may be substituted and the group may have 1 to 4 heteroatoms each independently selected from nitrogen, oxygen, and sulfur;
   n and m are each independently an integer from 0 to 2;
   each R⁴ and each R⁵ independently represent a halogen atom, CN, or a C₁-C₃ aliphatic group in which each hydrogen atom may be substituted; and
   R⁶ represents a hydrogen atom or a C₁-C₃ aliphatic group in which each hydrogen atom may be substituted], 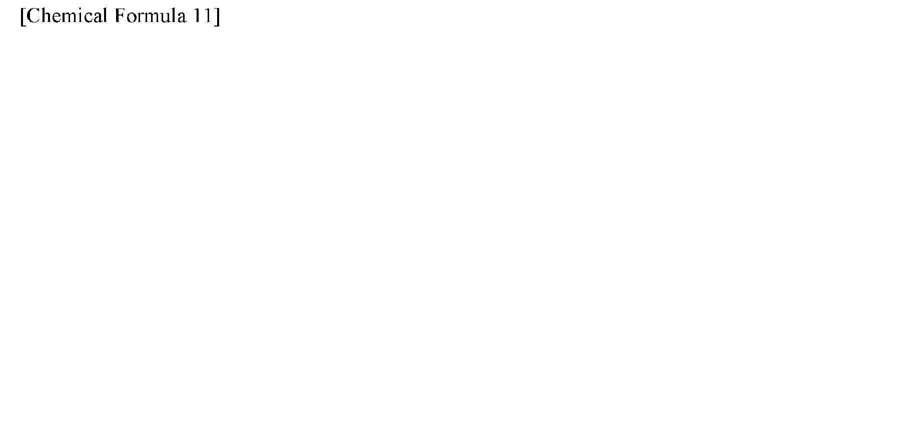 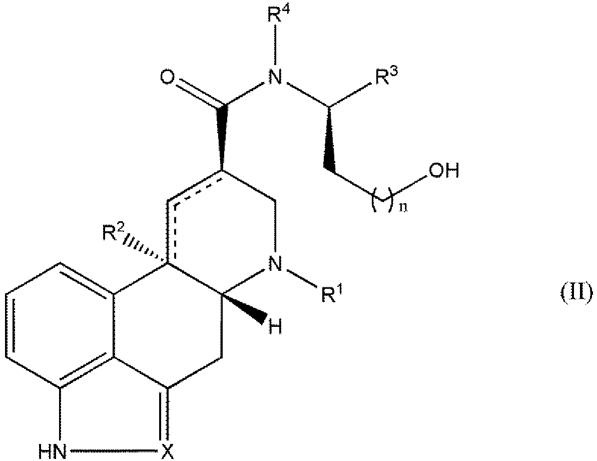 [in the formula (II),
      R¹ represents a hydrogen atom or a C₁-C₃ aliphatic group in which each hydrogen atom may be substituted;
      R² may or may not be present, and when present, R² represents a hydrogen atom;
      R³ and R⁴ each independently represent a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
      n is an integer from 0 to 2;
      X is a nitrogen atom or CZ [where Z is a hydrogen atom, a halogen atom, CN, or a C₁-C₃ aliphatic group in which each hydrogen atom may be substituted]; and
      a dotted line indicates the presence of a double bond at either position indicated by the dotted line].
[12] Use of a compound represented by the following formula (I) or (II), or a salt thereof for manufacture of a therapeutic or prophylactic medicament for Charcot-Marie-Tooth disease: 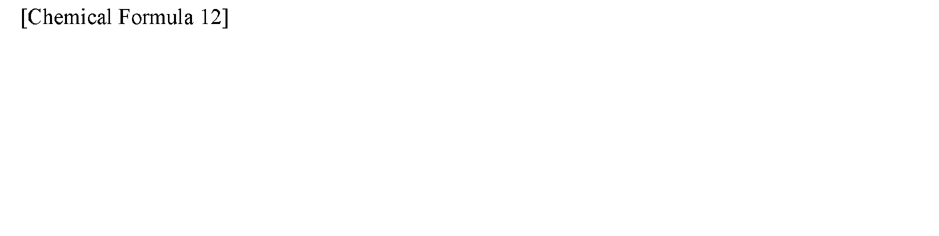 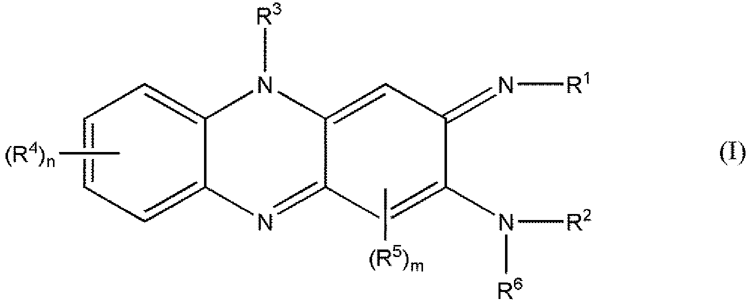 [in the formula (I),
   R¹ represents a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
   R² and R³ each independently represent a group that is selected from the group consisting of C₃-C₇ alicyclic hydrocarbon groups and 6- to 10-membered monocyclic or bicyclic aromatic groups, wherein each hydrogen atom in the group may be substituted and the group may have 1 to 4 heteroatoms each independently selected from nitrogen, oxygen, and sulfur;
   n and m are each independently an integer from 0 to 2;
   each R⁴ and each R⁵ each independently represent a halogen atom, CN, or a C₁-C₃ aliphatic group in which each hydrogen atom may be substituted; and
   R⁶ represents a hydrogen atom or a C₁-C₃ aliphatic group in which each hydrogen atom may be substituted], 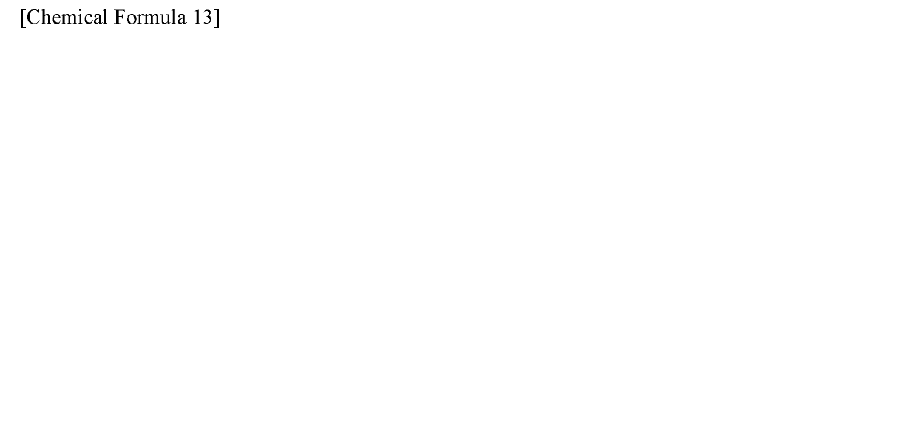 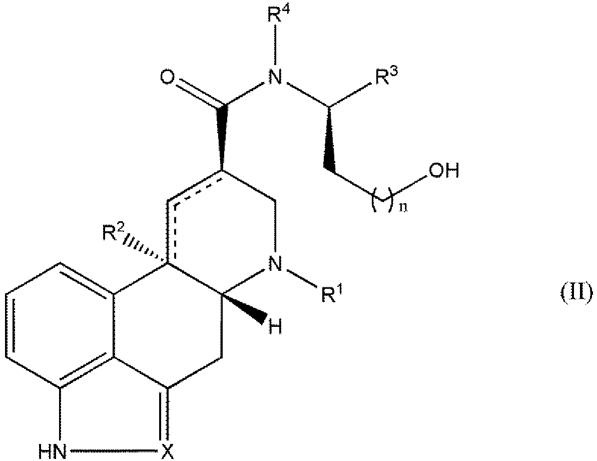 [in the formula (II),
      R¹ represents a hydrogen atom or a C₁-C₃ aliphatic group in which each hydrogen atom may be substituted;
      R² may or may not be present, and when present, R² represents a hydrogen atom;
      R³ and R⁴ each independently represent a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
      n is an integer from 0 to 2;
      X is a nitrogen atom or CZ [where Z is a hydrogen atom, a halogen atom, CN, or a C₁-C₃ aliphatic group in which each hydrogen atom may be substituted]; and
      a dotted line indicates the presence of a double bond at either position indicated by the dotted line].

### [ADVANTAGEOUS EFFECTS OF INVENTION]

The present invention enables treatment or prevention of CMT. Notably, since the present invention uses an existing medicament with confirmed safety as an active ingredient, it is expected that the present invention can shorten the development period required until clinical application.

### BRIEF DESCRIPTION OF DRAWINGS

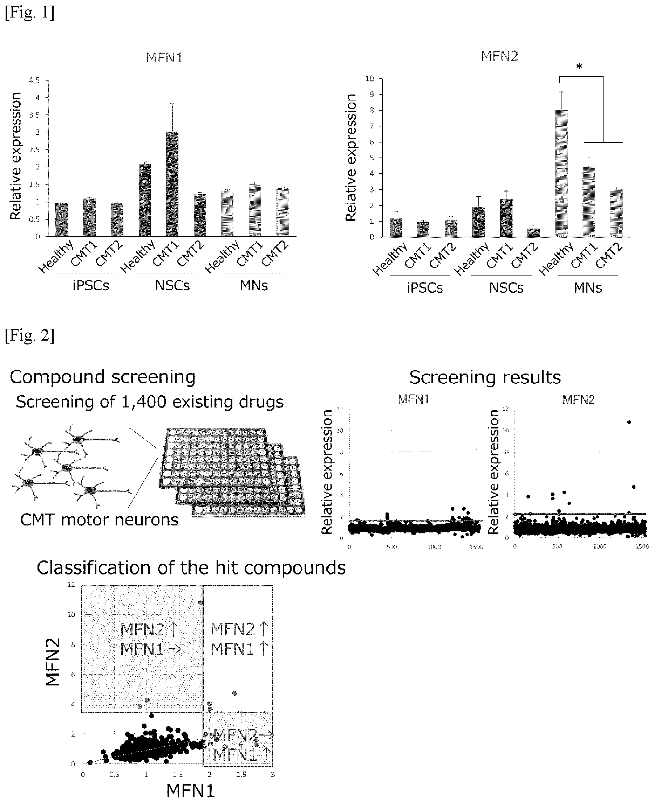

FIG. 1 shows the results of measurement of mRNA expression levels of MFN1 and MFN2 in iPS cells of healthy subjects, iPS cells of CMT patients with MFN2 mutations, neural stem cells derived from these iPS cells, and motor neurons derived from these iPS cells. The motor neurons derived from the iPS cells of the CMT patients exhibited a lower total mRNA expression level of MFN2 than the motor neurons derived from the iPS cells of the healthy subjects. Error bar: standard error, *: p < 0.05, N = 3 in each group.
FIG. 2 illustrates the overview of screening for compounds that increase the mRNA expression level of MFN1 or MFN2, performed using the motor neurons derived from iPS cells of CMT patients with MFN2 mutations, and also shows the screening results.

### DESCRIPTION OF EMBODIMENTS

As shown in examples to be described below, the inventors of the present invention found out that administration of clofazimine or methyl ergometrine maleate to cells increases the expression level of the mitofusin 1 (MFN1) gene in the cells. Since the symptoms of Charcot-Marie-Tooth disease (CMT) can be alleviated by increasing the expression level of the MFN1 gene in vivo, clofazimine or an analog thereof and methylergometrine or an analog thereof can be used for the treatment or prevention of CMT. Thus, the present invention provides a therapeutic or prophylactic medicament for CMT (hereinafter also referred to as "pharmaceutical of the present invention"), comprising clofazimine or an analog thereof or methylergometrine or an analog thereof (hereinafter also referred to as "compound of the present invention"). Unless otherwise stated, a therapeutic or prophylactic medicament (or method) for CMT encompasses a pharmaceutical (or method) capable of treating and preventing the disease.

The term "therapeutic medicament" as used herein shall be taken to encompass not only pharmaceuticals aimed at definitive therapy for CMT but also, for example, pharmaceuticals aimed at inhibiting the progression of CMT, pharmaceuticals aimed at alleviating the symptoms (for example, to the extent to achieve a state of minimal manifestation (MM) where the symptoms do not bother one's life or work activities), and pharmaceuticals for alleviating sequelae. For example, since CMT progresses over a long period of time (usually over one year), the progression of symptoms can be prevented by starting the treatment at an early stage. The term "prophylactic medicament" as used herein shall be taken to encompass not only pharmaceuticals aimed at reducing the risk of developing CMT in a subject who has not developed CMT but also pharmaceuticals aimed at reducing the risk of recurrence of CMT in a subject who has developed CMT. For example, it is also possible to prevent the development of CMT in a patient who potentially has a genetic background susceptible to CMT by administering the pharmaceutical of the present invention before the patient shows the symptoms of CMT. The same applies to the terms "method for treating" and "method for preventing".

As the pharmaceutical of the present invention, the compound of the present invention, which is an active ingredient, may be administered alone orally or parenterally, or alternatively, the compound of the present invention may be mixed with a pharmacologically acceptable carrier, excipient, diluent, and the like to form a pharmaceutical composition in a suitable dosage form and the thus-obtained pharmaceutical composition may be administered orally or parenterally. The pharmaceutical of the present invention can be administered to mammals (e.g., humans, rats, mice, guinea pigs, rabbits, sheep, horses, pigs, cows, dogs, cats, and monkeys). Thus, the present invention also provides a method for treating or preventing CMT in a mammal, comprising administering an effective amount of the compound of the present invention to the mammal.

CMT is a collective term for a group of hereditary diseases that cause both motor and sensory peripheral neuropathies. CMT to be treated or prevented by the present invention may be demyelinating-type CMT (type 1) (also referred to as "CMT1"), axonal-type CMT (type 2) (also referred to as "CMT2"), or other types of CMT, and preferably is CMT2. Examples of CMT1 include CMT type 1A (CMT1A) (causative gene: PMP22 gene), CMT type 1B (CMT1B) (causative gene: MPZ gene), CMT type 1C (CMT1C) (causative gene: LITAF gene), CMT type 1D (CMT1D) (causative gene: EGR2 gene), and CMT type 1E (CMT1E) (causative gene: PMP22 gene). Examples of CMT2 include CMT type 2A (CMT2A) (causative genes: MFN2 gene and KIF 1B gene), CMT type 2A2 (CMT2A2) (causative gene: MFN2 gene), CMT type 2B (CMT2B) (causative gene: RAB7 gene), CMT type 2C (CMT2C) (causative gene: TRPV4 gene), CMT type 2D (CMT2D) (causative gene: GARS gene), CMT type 2E (CMT2E) (causative gene: NEFL gene), CMT type 2F (CMT2F) (causative gene: HSPB 1 gene), CMT type 2G (CMT2G), CMT type 2H (CMT2H), CMT type 2I (CMT2I) (causative gene: MPZ gene), CMT type 2J (CMT2J) (causative gene: MPZ gene), CMT type 2K (CMT2K) (causative gene: GDAP1 gene), and CMT type 2L (CMT2L) (causative gene: HSPB8 gene). Examples of other types of CMT include CMT Type 3 (CMT3), CMT Type 4 (CMT4) (causative genes: PRX gene, NF-L gene, dyn2 gene, SOX10 gene, EGR2 gene, etc.), and X-linked CMT (CMTX) (causative genes: GJB 1 gene etc.).

In particular, CMT to be treated or prevented is preferably CMT2, of which CMT2A and CMT2A2 caused by mutations in the mitofusin 2 (MFN2) gene are more preferable. MFN2 is associated with mitochondrial fusion and mitochondrial axonal transport. Although a vast majority of mutations identified in patients with a family history of suffering CMT2A are in the GTPase domain, mutations of the MFN2 gene may be present outside this domain. Specifically, examples of the mutations in the MFN2 gene include the following mutations: conversion of histidine at position 128 in the amino acid sequence of MFN2, preferably substitution of histidine at position 128 with another amino acid, preferably with tyrosine (H128Y); and conversion of arginine at position 94, preferably substitution of arginine at position 94 with another amino acid, preferably with glutamine (R94Q), with glycine (R94G), or with tryptophan (R94W). Examples of other mutations in MFN2 include, but not limited to, V69F, L76P, T105M, H165D, I213T, F223L, T236M, V244M, P251A, V273G, R274Q, R280H, F284Y, K357N, R364P, R364W, R418X, E424G, and W740S (ZUchne S, et al. Nature Genet 36: 449-451 (2004), Kijima K, et al. Hum Genet 116: 23-27, (2005), Zhu D, Kennerson ML, et al. Neurology 65: 496-497 (2005), Lawson VH, et al. Neurology 65: 197-204 (2005), and Stuppia G, et al. J Neurol Sci 356(1-2): 7-18 (2015)).

In addition, the compound of the present invention can also be used as an agent for promoting MFN1 gene expression. Thus, in another aspect, the present invention provides an agent for promoting MFN1 gene expression comprising the compound of the present invention (hereinafter also referred to as "agent for promoting expression of the present invention"). As used herein, the agent for promoting expression of the present invention encompasses any agents as long as they promote the expression of the MFN1 gene at the cellular level or at the biological level. The term "gene expression" as used herein is intended to encompass at least "production of a functional protein encoded by mRNA of MFN1", but also further encompasses "production of mRNA of MFN1", unless otherwise stated. Accordingly, promotion of the expression of the MFN1 gene may encompass not only an increase in the abundance of a functional protein encoded by the gene, present in cells but also an increase in the abundance of mRNA transcribed from the gene, in the cells, as a result of administering the compound of the present invention.

The agent for promoting expression of the present invention is prepared in the form of a common pharmaceutical composition or pharmaceutical formulation or in the form of a cosmetic product or food, and administered orally or parenterally. The agent for promoting expression of the present invention can also be used as a reagent. The agent for promoting expression of the present invention can be administered to, for example, subjects including humans (e.g., mammals and cells, tissues, and organs of mammals). Thus, the present invention also provides a method for promoting the expression of the MFN1 gene in a subject, comprising administering the compound of the present invention to the subject.

Clofazimine or an analog thereof used in the present invention may be, for example, a compound represented by the following formula (I) or a salt thereof.

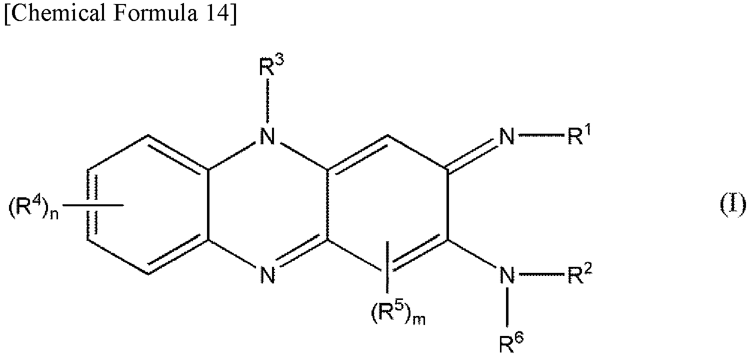

[In the formula (I),
R¹ represents a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
R² and R³ each independently represent a group that is selected from the group consisting of C₃-C₇ alicyclic hydrocarbon groups and 6- to 10-membered monocyclic or bicyclic aromatic groups, wherein each hydrogen atom in the group may be substituted and the group may have 1 to 4 heteroatoms each independently selected from nitrogen, oxygen, and sulfur;
n and m are each independently an integer from 0 to 2 (preferably n and m are both 0),
each R⁴ and each R⁵ independently represent a halogen atom, CN, or a C₁-C₃ aliphatic group in which each hydrogen atom may be substituted; and
R⁶ represents a hydrogen atom or a C₁-C₃ aliphatic group in which each hydrogen atom may be substituted].

In one embodiment, clofazimine or an analog thereof may be a compound represented by the formula (I), where R² and R³ are each independently a 6- to 10-membered monocyclic aromatic group in which each hydrogen atom may be substituted and that may have 1 to 4 heteroatoms each independently selected from nitrogen, oxygen and sulfur, or a salt thereof. In particular, it is preferable that R² and R³ in the formula (I) are both 6- to 10-membered (preferably 6-membered) monocyclic aromatic groups in which each hydrogen atom may be substituted. Also preferred is the above compound in which at least one of, preferably both of, the aromatic groups R² and R³ is an aromatic group in which at least one (preferably one) hydrogen atom is substituted with a halogen atom (preferably a chlorine atom), or a salt thereof.

In another embodiment, clofazimine or an analog thereof may be a compound represented by the formula (I), where R¹ is a C₁-C₆ aliphatic group in which each hydrogen atom may be substituted and that may have 1 to 4 (preferably 1) heteroatoms each independently selected from nitrogen, oxygen, and sulfur, or a salt thereof. Such a heteroatom is preferably a nitrogen atom. Substitution of a hydrogen atom is preferably substitution thereof with a C₁-C₃ aliphatic group (preferably alkyl group). Also preferred is the above compound in which R¹ in the formula (I) is a C₁-C₃ (preferably C₂-C₃) aliphatic group (preferably alkyl group), or a salt thereof.

Specific examples of clofazimine or an analogue thereof include compounds represented by the following formulae (I-1) to (I-10) and salts thereof. Of these, preferred is a compound represented by the following formula (I-1) (i.e., clofazimine; N,5-bis(4-chlorophenyl)-3-propan-2-yliminophenazin-2-amine) or a salt thereof.

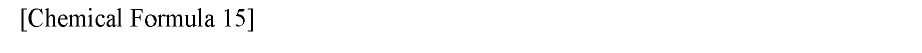

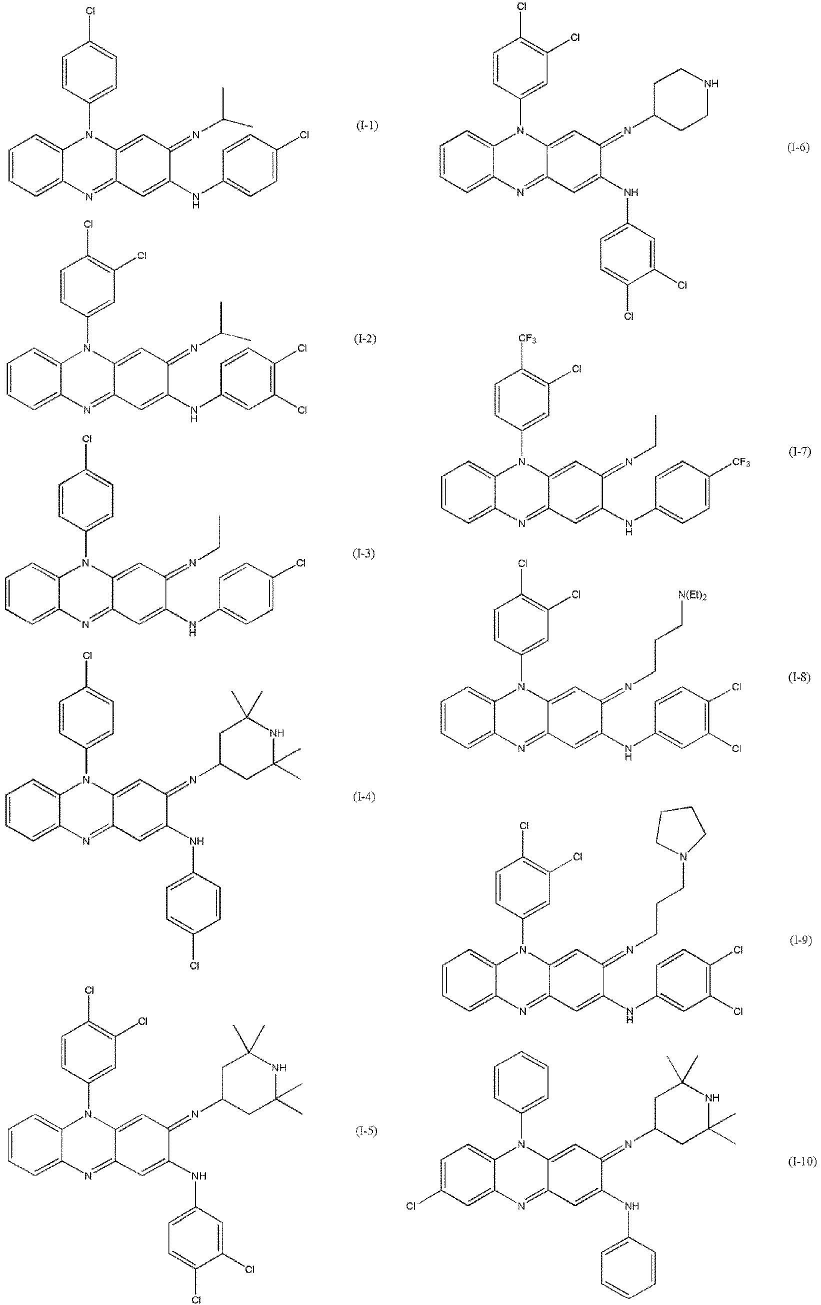

Methylergometrine or an analog thereof used in the present invention may be, for example, a compound represented by the following formula (II) or a salt thereof.

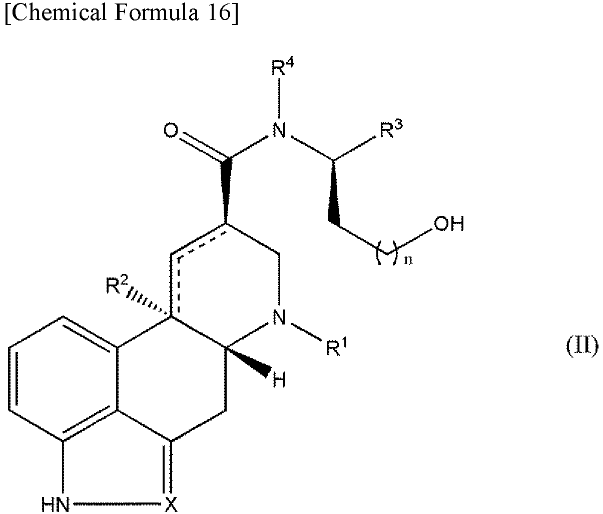

[In the formula (II),
R¹ represents a hydrogen atom or a C₁-C₃ (preferably C₁) aliphatic group (preferably alkyl group) in which each hydrogen atom may be substituted;
R² may or may not be present, and when present, R² represents a hydrogen atom;
R³ and R⁴ each independently represent a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
n is an integer from 0 to 2 (preferably 0);
X is a nitrogen atom or CZ [where Z is a hydrogen atom, a halogen atom, CN, or a C₁-C₃ aliphatic group in which each hydrogen atom may be substituted]; and
a dotted line indicates the presence of a double bond at either position indicated by the dotted line].

The above formula (II) can also be represented by the following formula (II-a) or (II-b).

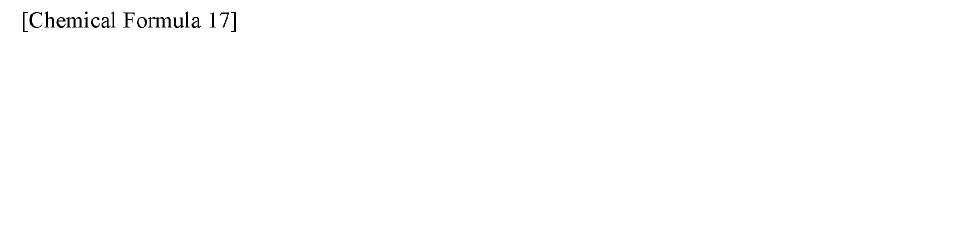

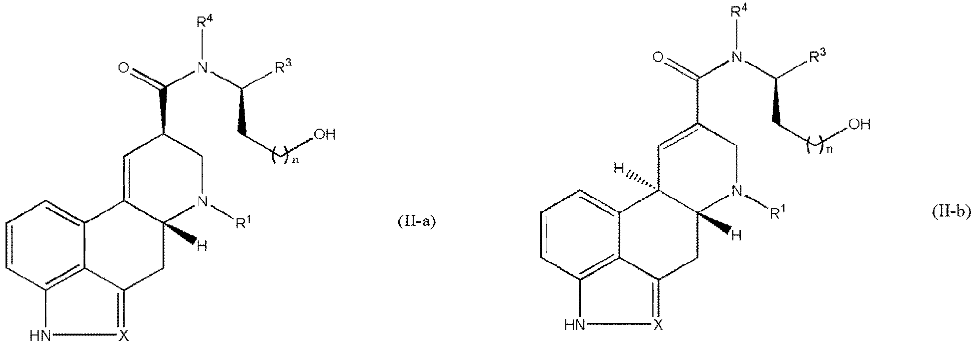

[In the formulae (II-a) and (II-b), the definitions of R¹, R³, R⁴, n, and X are the same as those in the formula (II)].

In one embodiment, methylergometrine or an analog thereof may be a compound represented by the formula (II), where X is CZ [where Z is a hydrogen atom, a halogen atom, or CN], or a salt thereof. In particular, preferred is the above compound in which Z is a hydrogen atom, or a salt thereof.

In another embodiment, methylergometrine or an analog thereof may be a compound represented by the above formula (II), where R³ and R⁴ each independently represent a hydrogen atom or a C₁-C₃ aliphatic group in which each hydrogen atom may be substituted, or a salt thereof. In particular, preferred is the above compound in which R³ is a C₁-C₃ (preferably C₂) aliphatic group (preferably alkyl group), or a salt thereof. Also preferred is the above compound in which R⁴ is a hydrogen atom, or a salt thereof. Accordingly, in a preferable embodiment, methylergometrine or an analog thereof may be the above compound in which R³ is a C₁-C₃ (preferably C₂) aliphatic group (preferably alkyl group) and R⁴ is a hydrogen atom, or a salt thereof.

Specific examples of methylergometrine or an analogue thereof include a compound represented by the following formula (II-1) (i.e., methylergometrine; (6aR,9R)-N-[(2S)-1-hydroxybutan-2-yl]-7-methyl-6,6a,8,9-tetrahydro-4H-indolo[4,3-fg]quinoline-9-carboxamide). Specific examples of a salt of the above compound include a salt represented by the following formula (II-2) (i.e., methylergometrine maleate; (6aR,9R)-N-((S)-1-hydroxybutan-2-yl)-7-methyl-4,6,6a,7,8,9-hexahydroindolo[4,3-fg]quinoline-9-carboxamide maleate).

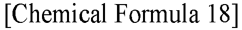

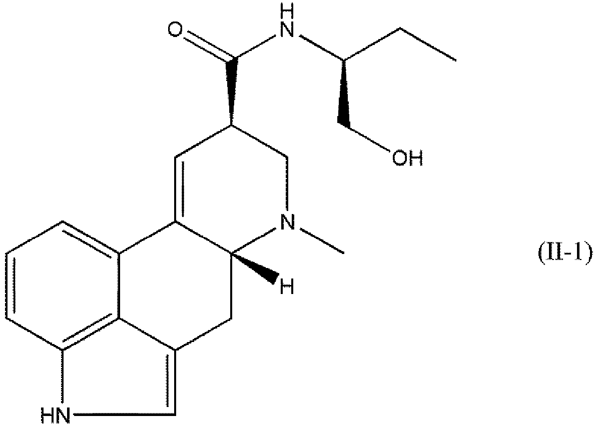

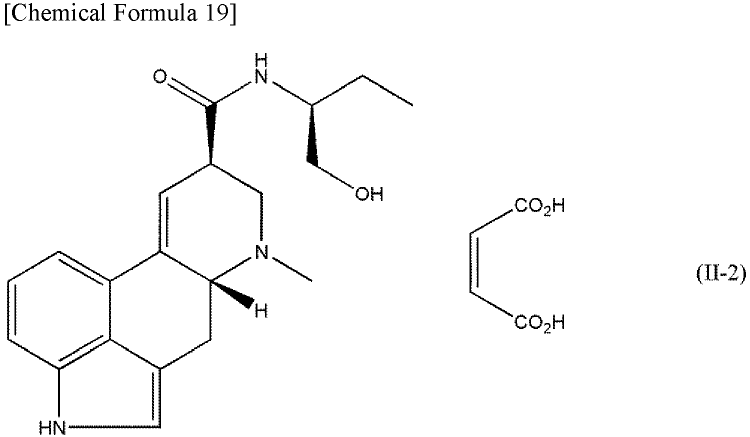

As used herein, the term "aliphatic group" means a straight or branched hydrocarbon chain that is fully saturated or contains one or more unsaturated bonds. Examples of the aliphatic group include straight or branched alkyl groups (e.g., methyl group, ethyl group, propyl group, isopropyl group, butyl group, 2-butyl group, 2-methylpropyl group, 1,1-dimethylethyl group, pentyl group, 3-pentyl group, 3-methylbutyl group, hexyl group, and 3-hexyl group), alkenyl groups (e.g., vinyl group, allyl group, 2-propynyl group, 2-butenyl group 3-methyl-2-butenyl group, and 3-hexenyl group), alkynyl groups (e.g., ethynyl group, 1-propynyl group, 2-propynyl group, 1-butynyl group, 2-butynyl group, 3-butynyl group, 1-pentynyl group, 2-pentynyl group, 3-pentynyl group, 4-pentynyl group, 1-hexynyl group, 2-hexynyl group, 3-hexynyl group, 4-hexynyl group, 5-hexynyl group, and 4-methyl-2-pentynyl group). The term "alicyclic hydrocarbon group" means a monocyclic or bicyclic hydrocarbon group that is fully saturated or contains one or more unsaturated bonds and that does not belong to aromatic hydrocarbon groups, or means an aliphatic group having such a hydrocarbon group. Examples of the alicyclic hydrocarbon group include cycloalkyl groups (e.g., cyclopropyl group, cyclobutyl group, cyclopentyl group, cyclohexyl group, and cycloheptyl group), cycloalkenyl groups (e.g., cyclopropenyl group, cyclobutenyl group, cyclopentenyl group, cyclohexenyl group, and cycloheptenyl group), (cycloalkyl)alkyl groups, (cycloalkenyl)alkyl groups, and (cycloalkyl)alkenyl groups.

The monocyclic or bicyclic aromatic group may be, for example, an aryl group or a heteroaryl group. Examples of the aryl group include aryl groups having 10 or less carbon atoms, such as a phenyl group and a naphthyl group. Examples of the heteroaryl group include 5- to 6-membered monocyclic groups containing 1 to 2 nitrogen atoms, 5- or 6-membered monocyclic groups containing 1 to 2 nitrogen atoms and one oxygen or sulfur atom, 5-membered monocyclic groups containing one oxygen or sulfur atom, and bicyclic groups that contain 1 to 4 nitrogen atoms and in which a 6-membered ring is fused with a 5- or 6-membered ring, and specific examples thereof include a 2-pyridyl group, 3-pyridyl group, 4-pyridyl group, 2-thienyl group, 3-thienyl group, 3-oxadiazolyl group, 1-imidazolyl group, 2-imidazolyl group, 2-thiazolyl group, 3-isothiazolyl group, 2-oxazolyl group, 3-isoxazolyl group, 2-furyl group, 3-furyl group, 3-pyrrolyl group, 8-quinolyl group, 2-quinazolinyl group, and 8-purinyl group.

Examples of the halogen atom include a fluorine atom, chlorine atom, bromine atom, and iodine atom.

As used herein, "each hydrogen atom may be substituted (or is substituted)" means that at least one of hydrogen atoms present in a group may be substituted (or is substituted) with another atom or group. That is, a group in which each hydrogen atom may be substituted (or is substituted) is, in other words, a group that may have (or has) a substituent. In the present specification, the substituent may be, for example, a halogen atom, cyano group, benzyloxy group, trifluoromethyl group, hydroxy group, lower alkoxy group, lower alkanoyloxy group, amino group, mono-lower alkylamino group, di-lower alkylamino group, carbamoyl group, lower alkylaminocarbonyl group, di-lower alkylaminocarbonyl group, lower alkoxycarbonylamino group, carboxyl group, lower alkoxycarbonyl group, lower alkylthio group, lower alkylsulfinyl group, lower alkylsulfonyl group, lower alkanoylamino group, lower alkylsulfonamide group, phthalimido group, heteroaryl group, aryl with a substituent(s) (substituted aryl), heteroaryl with a substituent(s) (substituted heteroaryl), saturated heterocyclic group, or group represented by the formula: -NR⁷R⁸ (where R⁷ and R⁸ each independently represent a hydrogen atom, lower alkoxy group, lower alkyl group, substituted lower alkyl group, cycloalkyl group, or aralkyl group, or R⁷ and R⁸ are bonded together and they represent, together with nitrogen atoms to which they are bound, a saturated cyclic amino group having 4 to 8 carbon atoms in its ring). Examples of the heteroaryl group are the same as those given above. Examples of the saturated heterocyclic group include 5- to 8-membered groups having one nitrogen atom, such as 1-piperidinyl and 1-pyrrolidinyl, 6- to 8-membered groups having two nitrogen atoms, and 6- to 8-membered groups having one nitrogen atom and one oxygen atom. The substituted alkyl group may be a C₁-C₆ alkyl group substituted with a cycloalkyl group or with a substituted cycloalkyl group, or an aralkyl group or substituted aralkyl group. Examples of the aralkyl group and substituted aralkyl group include C₁-C₆ alkyl groups substituted with the above-described aryl groups or a substituted aryl group, such as, for example, a benzyl group, 1-phenylethyl group, 2-phenylethyl group, and 2-naphthylmethyl group. In the present specification, an unsubstituted group is intended in the absence of the description "each hydrogen atom may be substituted", unless otherwise stated. In the present specification, the term "lower" means having 5 or less (preferably 3 or less) carbon atoms.

As clofazimine or an analog thereof or methylergometrine or an analog thereof, commercially available products may be used, or each of these compounds can be produced by a method known per se.

Clofazimine or an analog thereof can be produced according to the methods described in, for example, U.S. Patent No. 2,891,062, U.S. Patent No. 2,943,089, U.S. Patent No. 2,946,792, U.S. Patent No. 2,948,726, U.S. Patent No. 8,669,257, and literatures cited therein. Clofazimine is commercially available as a therapeutic medicament for Hansen's disease from Sandoz K.K. and other companies.

Methylergometrine or an analog thereof can be produced according to the methods described in, for example, U.S. Patent No. 3,920,664, U.S. Patent No. 4,201,862, U.S. Patent No. 5,480,885, and literatures cited therein. Methylergometrine is commercially available as a uterotonic medicament or a hemostatic agent from Mochida Pharmaceutical Co., Ltd., Fuji Pharma Co., Ltd., ASKA Pharmaceutical Co., Ltd., and other companies.

Clofazimine or an analog thereof or methylergometrine or an analog thereof shall be taken to encompass not only the free form thereof but also pharmacologically acceptable salts thereof. Examples of the pharmacologically acceptable salts, which vary depending on the types of compounds, include: base addition salts including inorganic base salts such as alkali metal salts (such as sodium salts and potassium salts), alkaline-earth metal salts (such as calcium salts and magnesium salts), aluminum salts, and ammonium salts and organic base salts such as trimethylamine, triethylamine, pyridine, picoline, ethanolamine, diethanolamine, triethanolamine, dicyclohexylamine, and N,N'-dibenzylethylenediamine; and acid addition salts including inorganic acid salts such as hydrochloride, hydrobromide, sulfate, hydroiodide, nitrate, and phosphate and organic acid salts such as citrate, oxalate, acetate, formate, propionate, benzoate, trifluoroacetate, maleate, tartrate, methanesulfonate, benzenesulfonate, and para-toluenesulfonate.

In the case where clofazimine or an analog thereof or methylergometrine or an analog thereof has isomers such as optical isomers, stereoisomers, regioisomers, and rotamers, any of the isomers and mixtures thereof are also encompassed in the compound of the present invention. For example, in the case where clofazimine or an analog thereof or methylergometrine or an analog thereof has optical isomers, an optical isomer separated from a racemate is also encompassed in the compound of the present invention. Each of these isomers can be obtained as a single product by a method known per se, such as a synthesis method, a separation method (e.g., concentration, solvent extraction, column chromatography, or recrystallization), or an optical resolution method (e.g., fractional crystallization, a chiral column method, or a diastereomer method).

Clofazimine or an analog thereof or methylergometrine or an analog thereof may be in the form of crystals, and regardless of whether it has only one crystal form or a mixture of crystal forms, it is encompassed in the compound of the present invention. Crystals can be produced by causing crystallization using a crystallization method known per se.

Clofazimine or an analog thereof or methylergometrine or an analog thereof may be a solvate (e.g., a hydrate) or an ansolvate (e.g., a non-hydrate), and both of them are encompassed in the compound of the present invention.

Compounds labeled with an isotope (e.g., ³H, ¹⁴C, ³⁵S, or ¹²⁵I) or the like are also encompassed in the compound of the present invention.

The composition for oral administration may be in a solid or liquid dosage form, and specific examples of the dosage form include tablets (including sugar-coated tablets and film-coated tablets), pills, granules, powdered drugs, capsules (including soft capsules), syrups, emulsions, and suspensions. On the other hand, the composition for parenteral administration may be, for example, an injection or a suppository, and the injection may encompass dosage forms such as an intravenous injection, subcutaneous injection, intradermal injection, intramuscular injection, and intravenous infusion. These formulations are produced by well-known methods using additives, examples of which include excipients (e.g., organic excipients including: sugar derivatives such as lactose, saccharose, glucose, mannitol, and sorbitol; starch derivatives such as corn starch, potato starch, α-starch, and dextrin; cellulose derivatives such as crystalline cellulose; gum arabic; dextran; and pullulan; and inorganic excipients including: silicate derivatives such as light anhydrous silicic acid, synthetic aluminum silicate, calcium silicate, and magnesium aluminometasilicate; phosphates such as calcium hydrogen phosphate; carbonates such as calcium carbonate; and sulfates such as calcium sulfate), lubricants (e.g., stearic acid and metal stearates such as calcium stearate and magnesium stearate; talc; colloidal silica; waxes such as beeswax and spermaceti; boric acid; adipic acid; sulfates such as sodium sulfate; glycol; fumaric acid; sodium benzoate; DL-leucine; lauryl sulfates such as sodium lauryl sulfate and magnesium lauryl sulfate; silicates such as silicic acid anhydrides and silicic acid hydrates; and the above-described starch derivatives), binders (e.g., hydroxypropylcellulose, hydroxypropylmethylcellulose, polyvinylpyrrolidone, macrogol, and the same compounds as those given above as examples of the excipients), disintegrants (e.g., cellulose derivatives such as low-substituted hydroxypropylcellulose, carboxymethylcellulose, carboxymethylcellulose calcium, and internally cross-linked sodium carboxymethylcellulose; and chemically modified starches and celluloses, such as carboxymethyl starch, sodium carboxymethyl starch, and cross-linked polyvinylpyrrolidone), emulsifiers (e.g., colloidal clays such as bentonite and Veegum; metal hydroxides such as magnesium hydroxide and aluminum hydroxide; anionic surfactants such as sodium lauryl sulfate and calcium stearate; cationic surfactants such as benzalkonium chloride; and non-ionic surfactants such as polyoxyethylene alkyl ethers, polyoxyethylene sorbitan fatty acid esters, and sucrose fatty acid esters), stabilizers (para-hydroxybenzoic acid esters such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid), taste and odor masking agents (e.g., commonly used sweeteners, acidulants, and flavors), and diluents.

The dose of the compound of the present invention, which is an active ingredient of the pharmaceutical or agent for promoting expression of the present invention, may vary according to various conditions including the type of compound and the symptoms, age, weight, and drug acceptability of a subject to which the compound is administered, and the compound can be administered to an adult one to six times a day with the lower limit per dose being 0.1 mg (suitably 0.5 mg) and the upper limit per dose being 1000 mg (suitably 500 mg) for oral administration and with the lower limit per dose being 0.01 mg (suitably 0.05 mg) and the upper limit per dose being 100 mg (suitably 50 mg) for parenteral administration. The dose may be increased or decreased in consideration of the symptoms. In particular, when compounds according to the present invention are compounds that have already been put on the market as pharmaceuticals for diseases other than the above-described disease, an appropriate dose can be selected for each compound within the range where the safety of the compound is confirmed.

The pharmaceutical or agent for promoting expression of the present invention can be used in combination with physical therapy and/or surgical therapy for CMT, or in combination with therapeutic or prophylactic medicaments for CMT (e.g., neurotrophic factors, progesterone inhibitors and stimulants, ascorbic acid, and curcumin). When any of these medicaments is used as a co-agent, such a co-agent may be formulated together with the compound of the invention and administered in the form of the thus-obtained single formulation, or alternatively, such a co-agent may be formulated separately from the compound of the present invention and the co-agent and the compound of the present invention may be administered simultaneously or sequentially through an administration route that is the same as or different from the administration route of the pharmaceutical or agent for promoting expression of the present invention. The dose of such a co-agent may be set to an amount normally used when it is administered alone, or may be set smaller than the amount normally used.

When the agent for promoting expression of the present invention is administered to cells, administration can typically be performed by culturing the cells in a culture medium comprising the agent for promoting expression of the present invention. Such culture can be carried out under conditions commonly used in the art, as long as the expression of the MFN1 gene can be promoted under such conditions.

The present invention will be described more specifically with reference to the following examples. It is to be noted, however, that the present invention is not limited to these examples by any means.

### EXAMPLES

### Materials and Methods

### Generation of iPS cells and cell culture

iPS cells established according to the method described in NPL 3 were used. The iPS cells were derived from peripheral-blood mononuclear cells (PBMCs) from CMT2 patients with MFN2 gene mutations (CMT1 (R94Q) and CMT2 (H128Y)) and healthy subjects without MFN2 mutations (Healthy). A feeder-free medium was used to perform maintenance culture of the iPS cells.

### Induction of neural stem cells (NSCs)

Human iPS cells were dissociated into single cells, which were added to a Matrigel-coated 24-well plate and cultured for 10 days with a 5% DFK medium (Dulbecco's modified Eagle's medium/Ham's F12; Thermo Fisher Scientific, Pittsburgh, PA), 5% KSR (Thermo Fisher Scientific), NEAA (Thermo Fisher Scientific), L-glutamine (Sigma-Aldrich), 0.1 M 2-ME (Thermo Fisher Scientific), 2 µM dorsomorphin, and 10 µM SB431542.

### Induction of motor neurons (MNs) by serum-free floating culture of embryoid body-like aggregates with quick reaggregation (SFEBq)

Human iPS cells were dissociated into single cells and quickly reaggregated in a U-bottom 96-well plate for resuspension culture (Greiner Bio-One, Frickenhausen, Germany), precoated with Pluronic (Sigma-Aldrich, St. Louis, MO) adjusted to a concentration of 2% using 100% ethanol. Aggregates of embryoid bodies were cultured at the neuronal induction stage (P1) for 12 days with a 5% DFK medium (Dulbecco's modified Eagle's medium/Ham's F12; Thermo Fisher Scientific, Pittsburgh, PA), 5% KSR (Thermo Fisher Scientific), NEAA (Thermo Fisher Scientific), L-glutamine (Sigma-Aldrich), 0.1 M 2-ME (Thermo Fisher Scientific), and 2 µM dorsomorphin. The aggregates were subjected to patterning in a Neurobasal medium (Thermo Fisher Scientific) supplemented with 2% vitamin A-free B27 (Thermo Fisher Scientific), 100 nM retinoic acid, 500 nM Smoothened agonist (Sonic Hedgehog signaling activator), and FGF 2 (12.5 ng/ml) (Thermo Fisher Scientific), and thereafter, the aggregates were attached to a culture vessel coated with Matrigel (Corning, Tewksbury, MA) on day 22.

Adherent embryoid bodies were cultured at the P2 stage in a neurobasal medium supplemented with a brain-derived neurotrophic factor (BDNF) (10 ng/ml), glial cell line-derived neurotrophic factor (GDNF) (10 ng/ml), and NT-3 (10 ng/ml). The embryoid bodies were detached from the culture vessel using Accutase (Innovative Cell Technologies, San Diego, CA) and dissociated into small clumps or single cells. In the maturation period P3, they were cultured in a 24-well culture vessel coated with Matrigel on day 35 at 500,000 cells per well.

### Measurement of MFN2 gene expression level

mRNA was extracted, and mRNA expression levels of MFN1 and MFN2 were quantified by qPCR.

### High-throughput screening

The overview of the screening method is shown in the upper left of FIG. 2. Constructs expressing Lhx3, Ngn2, and Isl1 under tetracycline induction were introduced into the iPS cells using PiggyBac vectors, whereby stable lines were generated. Doxycycline was added to the iPS cells, and the iPS cells were cultured for 7 days using a neuro-differentiation medium to generate motor neurons (Imamura et al. Sci Transl Med 2017).

The motor neurons generated from the iPS cells of the CMT patients with MFN2 mutations were cultured in 96-well plates, and about 1400 compounds were added thereto on day 10. On day 12, RNA was extracted, and qPCR was performed to evaluate the amounts of mRNAs of MFN1 and MFN2.

### Statistical analysis

All data are shown as mean ± standard error. Comparisons between groups were made using one-way or two-way ANOVA, followed by a post-hoc test using Scheffe's multiple comparison, p < 0.05 was considered statistically significant.

### Example 1: Analysis of MFN2 gene expression level in cells having mutated MFN2 gene

Relative MFN2 gene expression levels were measured in the iPS cells, the neural stem cells, and the motor neurons. The results are shown in FIG. 1. As can be seen in FIG. 1, the motor neurons having the mutated MFN2 gene exhibited a decrease in MFN2 gene expression level. On this account, screening was performed to search for substances that promote the expression of the MFN2 gene or substances that promote the expression of the MFN1 gene (it has been reported that overexpression of the MFN1 gene in CMT model mice alleviates the symptoms of CMT).

### Example 2: Selection of candidate substances

Screening was performed to search for existing drugs that increase the expression levels of the MFN1 gene or MFN2 gene using the motor neurons having the mutated MFN2 gene. The result of the screening is shown in the upper right of FIG. 2. The result of classification of the hit compounds is shown in the lower left of FIG. 2. Drugs that act on the cardiovascular system and anticancer drugs were excluded from the hit compounds, and the following two existing drugs currently used for the treatment of the brain and nervous system were selected. The expression levels of the MFN1 gene and the MFN2 gene are expressed as relative values with the mRNA expression levels thereof when a vehicle (DMSO) was administered being defined as 1.

### (1) Clofazimine

Relative expression level of the MFN1 gene: 1.888976
Relative expression level of the MFN2 gene: 1.196658

### (2) Methylergometrine maleate

Relative expression level of the MFN1 gene: 2.006676
Relative expression level of the MFN2 gene: 3.675887

These results indicate that clofazimine can promote the expression of the MFN1 gene and methylergometrine maleate can promote the expression of the MFN1 gene and the MFN2 gene. Accordingly, it is strongly suggested that these compounds can exhibit a therapeutic or preventive effect on Charcot-Marie-Tooth disease.

### INDUSTRIAL APPLICABILITY

Clofazimine or an analog thereof and methylergometrine or an analog thereof are useful for the treatment or prevention of Charcot-Marie-Tooth disease. Notably, drugs that have already been put on the market as pharmaceuticals for other diseases have potential for allowing rapid development of pharmaceuticals that can treat or prevent Charcot-Marie-Tooth disease at low cost, because clinical and non-clinical data on safety and other matters have been accumulated for these drugs and libraries of neighboring compounds already exist.

The present application claims priority based on Japanese Patent Application No. 2022-045440 filed in Japan (filing date: March 22, 2022), the disclosure of which is incorporated herein in its entirety by reference.

## Claims

1. A therapeutic or prophylactic medicament for Charcot-Marie-Tooth disease, comprising:
a compound represented by the following formula (I) or (II), or a salt thereof: 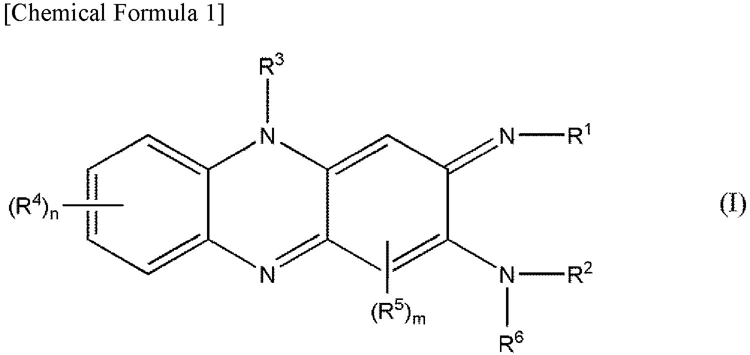 [in the formula (I),
R¹ represents a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
R² and R³ each independently represent a group that is selected from the group consisting of C₃-C₇ alicyclic hydrocarbon groups and 6- to 10-membered monocyclic or bicyclic aromatic groups, wherein each hydrogen atom in the group may be substituted and the group may have 1 to 4 heteroatoms each independently selected from nitrogen, oxygen, and sulfur;
n and m are each independently an integer from 0 to 2;
each R⁴ and each R⁵ independently represent a halogen atom, CN, or a C₁-C₃ aliphatic group in which each hydrogen atom may be substituted; and
R⁶ represents a hydrogen atom or a C₁-C₃ aliphatic group in which each hydrogen atom may be substituted], 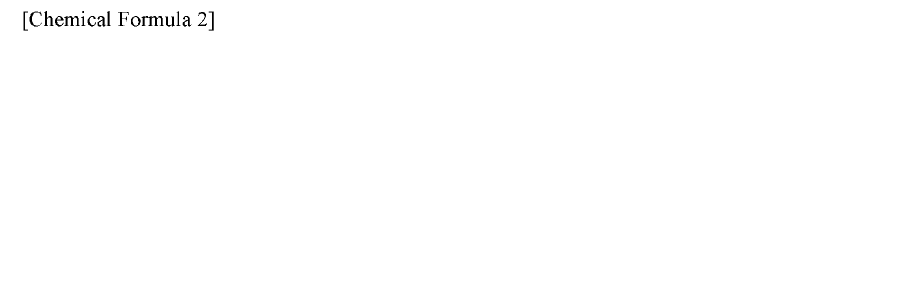 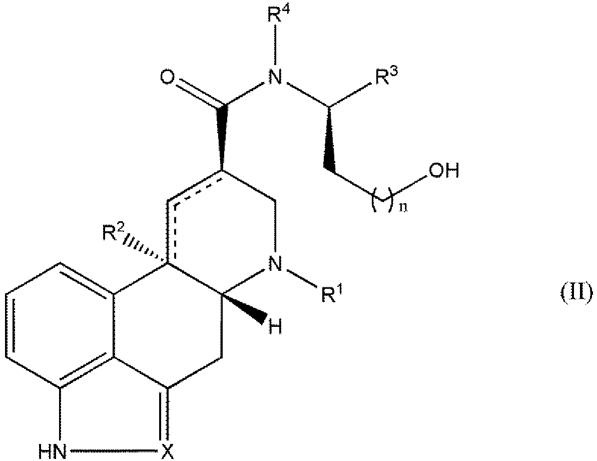 [in the formula (II),
R¹ represents a hydrogen atom or a C₁-C₃ aliphatic group in which each hydrogen atom may be substituted;
R² may or may not be present, and when present, R² represents a hydrogen atom;
R³ and R⁴ each independently represent a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
n is an integer from 0 to 2;
X is a nitrogen atom or CZ [where Z is a hydrogen atom, a halogen atom, CN, or a C₁-C₃ aliphatic group in which each hydrogen atom may be substituted]; and
a dotted line indicates the presence of a double bond at either position indicated by the dotted line].

2. The therapeutic or prophylactic medicament according to claim 1, wherein R² and R³ in the formula (I) are each independently a 6- to 10-membered monocyclic aromatic group in which each hydrogen atom may be substituted and that may have 1 to 4 heteroatoms each independently selected from nitrogen, oxygen, and sulfur.

3. The therapeutic or prophylactic medicament according to claim 1 or 2, wherein R¹ in the formula (I) is a C₁-C₆ aliphatic group in which each hydrogen atom may be substituted.

4. The therapeutic or prophylactic medicament according to any one of claims 1 to 3, wherein the formula (I) is represented by the following formula (I-1):

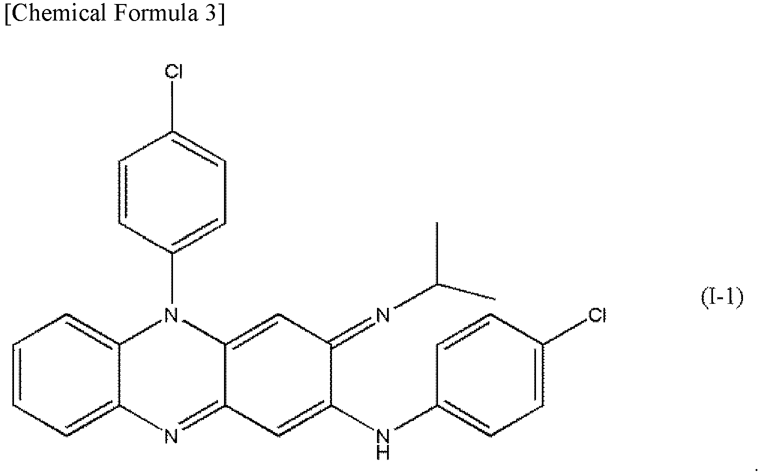

5. The therapeutic or prophylactic medicament according to claim 1, wherein X in the formula (II) is CZ [where Z represents a hydrogen atom, a halogen atom, or CN].

6. The therapeutic or prophylactic medicament according to claim 1 or 5, wherein R³ and R⁴ in the formula (II) are each independently a hydrogen atom or a C₁-C₃ aliphatic group in which each hydrogen atom may be substituted.

7. The therapeutic or prophylactic medicament according to any one of claims 1, 5, and 6, wherein the formula (II) is represented by the following formula (II-1):

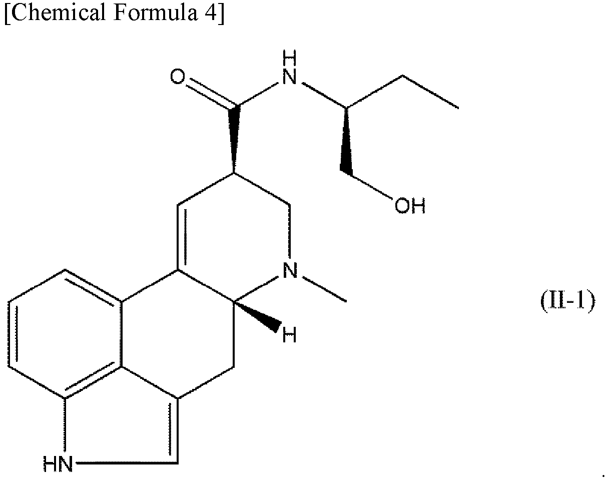

8. The therapeutic or prophylactic medicament according to any one of claims 1 to 7, wherein the Charcot-Marie-Tooth disease is Charcot-Marie-Tooth disease type 2.

9. An agent for promoting mitofusin 1 gene expression, comprising:
a compound represented by the following formula (I) or (II), or a salt thereof: 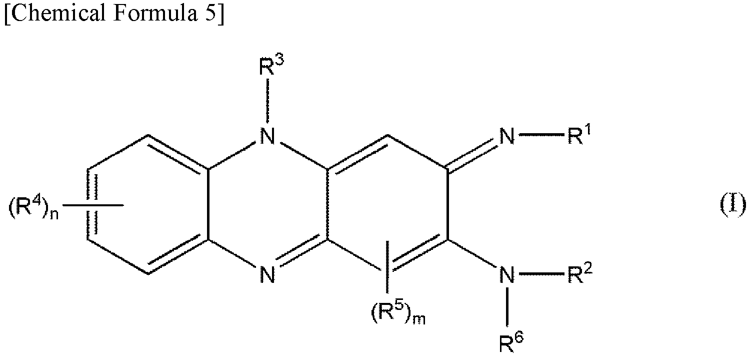 [in the formula (I),
R¹ represents a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
R² and R³ each independently represent a group that is selected from the group consisting of C₃-C₇ alicyclic hydrocarbon groups and 6- to 10-membered monocyclic or bicyclic aromatic groups, wherein each hydrogen atom in the group may be substituted and the group may have 1 to 4 heteroatoms each independently selected from nitrogen, oxygen, and sulfur;
n and m are each independently an integer from 0 to 2;
each R⁴ and each R⁵ independently represent a halogen atom, CN, or a C₁-C₃ aliphatic group in which each hydrogen atom may be substituted; and
R⁶ represents a hydrogen atom or a C₁-C₃ aliphatic group in which each hydrogen atom may be substituted], 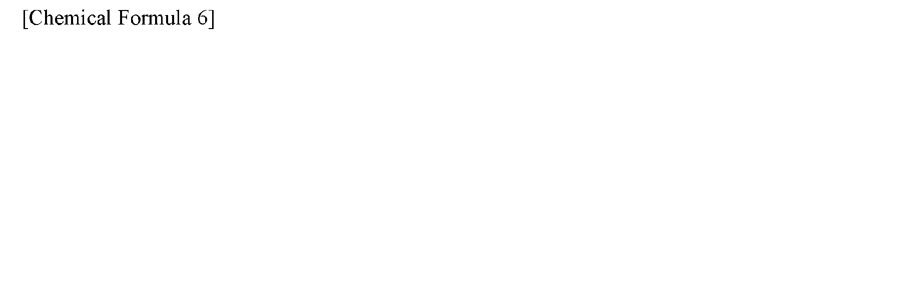 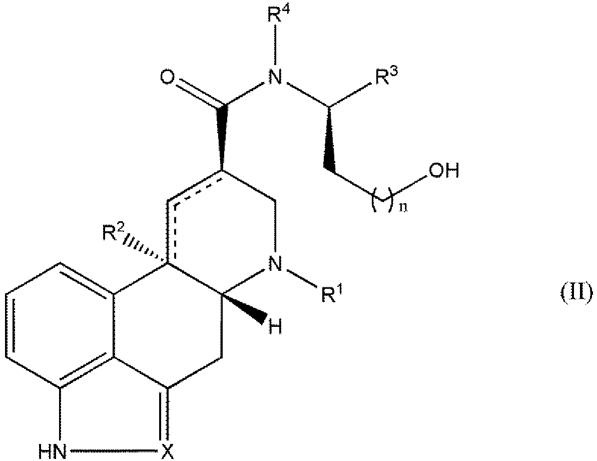 [in the formula (II),
R¹ represents a hydrogen atom or a C₁-C₃ aliphatic group in which each hydrogen atom may be substituted;
R² may or may not be present, and when present, R² represents a hydrogen atom;
R³ and R⁴ each independently represent a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur;
n is an integer from 0 to 2;
X is a nitrogen atom or CZ [where Z is a hydrogen atom, a halogen atom, CN, or a C₁-C₃ aliphatic group in which each hydrogen atom may be substituted]; and
a dotted line indicates the presence of a double bond at either position indicated by the dotted line].
